(19) Europäisches Patentamt / European Patent Office / Office européen des brevets

(11) **EP 4 159 237 A1**

(12) **EUROPEAN PATENT APPLICATION**
published in accordance with Art. 153(4) EPC

(43) Date of publication:
**05.04.2023 Bulletin 2023/14**

(21) Application number: **21813986.3**

(22) Date of filing: **28.05.2021**

(51) International Patent Classification (IPC):
*A61K 39/395* (2006.01)  *A61P 29/00* (2006.01)
*A61P 35/00* (2006.01)  *A61P 43/00* (2006.01)
*C12N 5/10* (2006.01)  *C12N 1/15* (2006.01)
*C12N 1/19* (2006.01)  *C12N 1/21* (2006.01)
*C12N 15/12* (2006.01)  *C12N 15/57* (2006.01)
*C12N 15/63* (2006.01)  *A61K 38/48* (2006.01)
*C12N 9/64* (2006.01)  *A61K 35/12* (2006.01)

(52) Cooperative Patent Classification (CPC):
**A61K 35/12; A61K 39/395; A61P 35/00;
C07K 14/435; C12N 5/10; C12N 9/48; C12N 9/64;
C12N 15/63; C12N 15/74; C12N 15/80;
C12N 15/81;** A61K 38/48; A61P 29/00; A61P 43/00

(86) International application number:
**PCT/JP2021/020334**

(87) International publication number:
**WO 2021/241719 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.05.2020 JP 2020093777**

(71) Applicant: **CHUGAI SEIYAKU KABUSHIKI KAISHA
Tokyo 115-8543 (JP)**

(72) Inventors:
• **KOMORI, Yasunori
Gotemba-shi, Shizuoka 412-8513 (JP)**

• **IGAWA, Tomoyuki
Synapse, 138623 (SG)**
• **NARITA, Atsushi
Tokyo 103-8324 (JP)**
• **ISHII, Shinya
Kamakura-shi, Kanagawa 247-8530 (JP)**

(74) Representative: **Vossius & Partner
Patentanwälte Rechtsanwälte mbB
Siebertstraße 3
81675 München (DE)**

Remarks:
The complete document including Reference Table(s) and the Sequence Listing(s) can be downloaded from the EPO website

(54) **IMPROVED GRANZYME B VARIANT**

(57) The present invention relates to granzyme B variants with increased protease activities and/or increased resistance against inhibitors; polynucleotides encoding the granzyme B variants; cells expressing the granzyme B variants; pharmaceutical compositions containing cells expressing the granzyme B variants; and pharmaceutical compositions containing the granzyme B variants. In some embodiments, the pharmaceutical compositions may be used in combination with cells expressing chimera receptors and/or antigen-binding molecules.

EP 4 159 237 A1

**Description**

[Technical Field]

The present invention relates to granzyme B variants with increased protease activities and/or increased resistance against inhibitors; polynucleotides encoding the granzyme B variants; cells expressing the granzyme B variants; pharmaceutical compositions comprising cells expressing the granzyme B variants; and pharmaceutical compositions comprising the granzyme B variants.

[Background Art]

Granzymes are proteases including a group of family members. Granzymes are expressed in T cells and NK cells - cytotoxic lymphocytes - and when these cells recognize a target antigen, granzymes are secreted to target cells (cells expressing the target antigen) together with perforin and such to exert cytotoxic activity. After transferring into the target cells, granzymes cleave various substrates such as BID and caspases and finally activate signal transduction systems that are involved in apoptosis in the target cells, thereby inducing cell death of the target cells (NPL 1).

Of the granzyme family, granzyme A and granzyme B have been suggested to make a particularly large contribution to cytotoxic activity. While granzyme A functions by forming a dimer, granzyme B functions as a monomer. Thus, it is easy to produce, express, and purify variants of granzyme B using protein engineering, and research and development has been performed with a view to drug discovery. For example, various variants including granzyme B with an scFv that binds to a tumor-associated antigen fused thereto are reported (NPL 2).

On the other hand, there is a limit to the use of granzyme B as an anti-tumor agent. The reasons for this include the limited protease activity of natural granzyme B and suppression of granzyme B activity by the development of inhibitors to granzyme B in target cells. NPL 3 shows that PI-9, an inhibitor of granzyme B activity, is highly expressed in tumors. Moreover, NPL 4 shows that heparin, whose production is known to be enhanced in tumors, inhibits granzyme B activity. That is, in tumors in which these inhibitors are highly expressed, it is considered that granzyme B, even if it is allowed to act on such tumors, may not exert sufficient cytotoxic activity and thus has only limited antitumor activity.

As granzyme variants that have resistance to these inhibitors, PI-9-resistant (PTL 1 and NPL 5) and heparin-resistant (NPL 6) granzyme B variants have been reported in previous studies. Furthermore, there is a report of granzyme B variants (PTL 2) in which comprehensive alterations were made to rat granzyme B to cleave VEGF and VEGF receptors.

On the other hand, when cytotoxic substances such as granzymes are used for therapy, it is preferable that they exert cytotoxic effects specifically on target cells (e.g., tumor cells). As an example of techniques for achieving this, there is a report on a method that uses transgenic cells that are transfected with a gene encoding a wild-type granzyme or the like and a gene encoding a chimeric antigen receptor (CAR) such that the cells are activated by the binding of the CAR to a target antigen and come to express the granzyme or the like at an increased level (PTL 3).

[Citation List]

[Patent Literature]

[PTL 1] US9528101B
[PTL 2] WO2005100556A2
[PTL 3] Japanese Patent Application *Kohyo* Publication No. (JP-A) 2019-526285 (unexamined Japanese national phase publication corresponding to a non-Japanese international publication)

[Non-Patent Literature]

[NPL 1] How Do Cytotoxic Lymphocytes Kill Cancer Cells?

Luis Martinez-Lostao, Alberto Anel and Julian Pardo
Clin Cancer Res. 2015 Nov 15; 21(22): 5047-56

[NPL 2] Delivery and therapeutic potential of human granzyme B

Kurschus FC1, Jenne DE.
Immunol Rev. 2010 May; 235(1): 159-71

[NPL 3] Blockade of the granzyme B/perforin pathway through overexpression of the serine protease inhibitor PI-9/SPI-6 constitutes a mechanism for immune escape by tumors J. P. Medema, J. de Jong, L. T. C. Peltenburg, E. M. E. Verdegaal, A. Gorter, S. A. Bres, K. L. M. C. Franken, M. Hahne, J. P. Albar, C. J. M. Melief, and R. OffringaProc Natl Acad Sci USA. 2001 Sep 25; 98(20): 11515-11520
[NPL 4] Cationic Sites on Granzyme B Contribute to Cytotoxicity by Promoting Its Uptake into Target Cells

Catherina H. Bird, Jiuru Sun, Kheng Ung, Diana Karambalis, James C. Whisstock, Joseph A. Trapani, and Phillip I. Bird
Mol Cell Biol. 2005 Sep; 25(17): 7854-7867

[NPL 5] Design of human granzyme B variants resistant to serpin B9

Losasso V, Schifer S, Barth S, Carloni P.
Proteins. 2012 Nov; 80(11): 2514-22

[NPL 6] Granzyme B delivery via perforin is restricted by size, but not by heparan sulfate-dependent endocytosis

Kurschus FC, Fellows E, Stegmann E, Jenne DE.
Proc Natl Acad Sci USA. 2008 Sep 16; 105(37): 13799-13804

[Summary of Invention]

[Technical Problem]

The granzyme B variants reported in PTL 1 show minor enhancement of protease activity compared to human wild-type granzyme B, and may not have sufficient cytotoxic activity. In PTL 2, while comprehensive alterations were made to rat granzyme B, there is no disclosure of data on resistance to inhibitors, and it is unclear whether these variants exert sufficient cytotoxic activity in the presence of inhibitors.

Furthermore, previous studies have proposed methods of using purified granzyme B or its variants by direct intravenous injection and so on. In such methods of use, however, there is a risk that cells which have taken up the administered granzyme B may undergo cell death and cause serious side effects. Accordingly, there is also a need to study a technique other than direct injection, as a therapeutic method using granzyme B.

[Solution to Problem]

Based on the above problems, the present inventors aimed to increase the activity of granzyme B, by discovering multiple alterations that increase protease activity through comprehensive alterations and by combining the alterations. As a result, the present inventors found that the activity of granzyme B protease is enhanced by genetic alterations that have not been reported in prior literatures. Furthermore, the present inventors showed that combining those alterations can significantly increase the activity relative to that of wild-type granzyme B. Moreover, the present inventors examined the resistance of the variants to granzyme B inhibitors to show that the variants exhibit high protease activity even in the presence of such inhibitors.

Thus, the present disclosure provides granzyme B variants whose protease activity and/or resistance to inhibitors has been enhanced by genetic alterations, and further provides, as medical uses of the granzyme B variants, pharmaceutical compositions comprising such a variant, pharmaceutical compositions comprising a cell expressing such a variant, and pharmaceutical compositions comprising such a variant used in combination with a receptor and/or a therapeutic antibody.

The present disclosure is based on the above findings, and specifically encompasses the embodiments exemplarily described below.

[1] A granzyme B variant comprising one or more amino acid residues selected from 1) to 20) below:

1) T, E, N, or V at position 43; 2) L or F at position 44; 3) Q, L, or A at position 45; 4) I, E, F, or Q at position 46; 5) V at position 47; 6) F or K at position 48; 7) L at position 99; 8) A at position 106; 9) M at position 149; 10) L at position 151; 11) P at position 155; 12) L at position 172; 13) Q, E, or I at position 175; 14) P at position 183;

15) L at position 184; 16) R at position 200; 17) I at position 217; 18) F at position 219; 19) G or S at position 222; and 20) V at position 229.

[2] The granzyme B variant of [1], wherein the variant comprises any one of the combinations of amino acid residues 1) to 12) below:

1) L at position 44, K at position 48, P at position 155, L at position 172, I at position 175, and R at position 200;
2) L at position 44, E at position 48, P at position 155, L at position 172, I at position 175, and R at position 200;
3) F at position 44, K at position 48, P at position 155, L at position 172, I at position 175, and R at position 200;
4) F at position 44, E at position 48, P at position 155, L at position 172, I at position 175, and R at position 200;
5) L at position 44, I at position 46, P at position 155, L at position 172, I at position 175, and R at position 200;
6) L at position 44, E at position 46, P at position 155, L at position 172, I at position 175, and R at position 200;
7) L at position 44, F at position 46, P at position 155, L at position 172, I at position 175, and R at position 200;
8) L at position 44, Q at position 46, P at position 155, L at position 172, I at position 175, and R at position 200;
9) F at position 44, I at position 46, P at position 155, L at position 172, I at position 175 and R at position 200;
10) F at position 44, E at position 46, P at position 155, L at position 172, I at position 175, and R at position 200;
11) F at position 44, F at position 46, P at position 155, L at position 172, I at position 175, and R at position 200; and
12) F at position 44, Q at position 46, P at position 155, L at position 172, I at position 175, and R at position 200.

[3] The granzyme B variant of [1] or [2], wherein protease activity is enhanced more than that of human wild-type granzyme B.
[4] The granzyme B variant of any one of [1] to [3], which has resistance to an inhibitor to human wild-type granzyme B.
[5] The granzyme B variant of [4], wherein the inhibitor is PI-9 or heparin.
[6] An isolated nucleic acid encoding the granzyme B variant of any one of [1] to [5].
[7] A vector comprising the isolated nucleic acid of [6].
[8] A cell transformed or transduced with the isolated nucleic acid of [6] or the vector of [7].
[9] A cell expressing the granzyme B variant of any one of [1] to [5].
[10] A pharmaceutical composition comprising the isolated nucleic acid of [6], the vector of [7], or the cell of [8] or [9].
[11] A pharmaceutical composition comprising the granzyme B variant of any one of [1] to [5].
[12] A pharmaceutical composition for use in combination with administration of a cell expressing a receptor, wherein the composition comprises a granzyme B variant or a cell expressing a granzyme B variant,

wherein the receptor activates the cell expressing the receptor by its binding to a ligand, and
wherein the granzyme B variant has enhanced protease activity more than that of human wild-type granzyme B and has resistance to an inhibitor to human wild-type granzyme B.

[13] The pharmaceutical composition of [12], wherein the receptor is a chimeric receptor which comprises an extracellular binding domain, a transmembrane domain, and an intracellular signaling domain, and binds to the ligand *via* the extracellular binding domain.
[14] The pharmaceutical composition of [12], wherein the receptor is a T-cell receptor whose ligand is a neoantigen.
[15] The pharmaceutical composition of any one of [12] to [14], wherein the granzyme B variant is the granzyme B variant of [1], [2], or [5].
[16] The pharmaceutical composition of any one of [12] to [15], which comprises the cell expressing the receptor.
[17] The pharmaceutical composition of [16], wherein the receptor and the granzyme B variant are expressed in the same T cell.
[18] A pharmaceutical composition for use in combination with administration of an antigen-binding molecule and administration of a cell expressing a chimeric receptor, wherein the composition comprises a granzyme B variant or a cell expressing a granzyme B variant,

wherein the antigen-binding molecule has an ability to bind to a target antigen,
wherein the chimeric receptor comprises an extracellular binding domain, a transmembrane domain, and an intracellular signaling domain and is capable of binding to a cell expressing the target antigen *via* the binding of the extracellular binding domain to the antigen-binding molecule, and
wherein the granzyme B variant has enhanced protease activity more than that of human wild-type granzyme B and has resistance to an inhibitor to human wild-type granzyme B.

[19] The pharmaceutical composition of [18],

wherein the antigen-binding molecule comprises a linker that is cleavable by a protease, and
wherein the extracellular binding domain is capable of binding to the antigen-binding molecule after cleavage of the linker.

[20] The pharmaceutical composition of [18] or [19], wherein the granzyme B variant is the granzyme B variant of [1], [2], or [5].

[21] The pharmaceutical composition of any one of [18] to [20], which comprises the cell expressing the chimeric receptor.

[22] The pharmaceutical composition of [21], wherein the chimeric receptor and the granzyme B variant are expressed in the same T cell.

[23] The pharmaceutical composition of any one of [13] and [15] to [22], wherein the chimeric receptor is a chimeric antigen receptor.

[A1] A granzyme B variant comprising one or more amino acid residue mutations selected from 1) to 20) below relative to human wild-type granzyme B:

1) 43T, 43E, 43N, or 43V; 2) 44L or 44F; 3) 45Q, 45L, or 45A; 4) 46I, 46E, 46F, or 46Q; 5) 47V; 6) 48F or K; 7) 99L; 8) 106A; 9) 149M; 10) 151L; 11) 155P; 12) 172L; 13) 175Q, 175E, or 175I; 14) 183P; 15) 184L; 16) 200R; 17) 217I; 18) 219F; 19) 222G or 222S; and 20) 229V.

[A2] The granzyme B variant of [A1], which comprises one or more amino acid residue mutations selected from 1) to 20) below relative to human wild-type granzyme B:

1) Q43T, Q43E, Q43N, or Q43V; 2) K44L or K44F; 3) S45Q, S45L, or S45A; 4) L46I, L46E, L46F, or L46Q; 5) K47V; 6) R48F; 7) A99L; 8) S106A; 9) Q149M; 10) A151L; 11) K155P; 12) K172L; 13) S175Q, S175E, or S175I; 14) S183P; 15) T184L; 16) K200R; 17) V217I; 18) Y219F; 19) N222G or N222S; and 20) A229V.

[A3] The granzyme B variant of [A1], wherein the amino acid residue mutations are any of 1) to 12) below:

1) 44L, 48K, 155P, 172L, 175I, and 200R;
2) 44L, 48E, 155P, 172L, 175I, and 200R;
3) 44F, 48K, 155P, 172L, 175I, and 200R;
4) 44F, 48E, 155P, 172L, 175I, and 200R;
5) 44L, 46I, 155P, 172L, 175I, and 200R;
6) 44L, 46E, 155P, 172L, 175I, and 200R;
7) 44L, 46F, 155P, 172L, 175I, and 200R;
8) 44L, 46Q, 155P, 172L, 175I, and 200R;
9) 44F, 46I, 155P, 172L, 175I, and 200R;
10) 44F, 46E, 155P, 172L, 175I, and 200R;
11) 44F, 46F, 155P, 172L, 175I, and 200R; and
12) 44F, 46Q, 155P, 172L, 175I, and 200R.

[A4] The granzyme B variant of [A3], wherein the amino acid residue mutations are any of 1) to 12) below:

1) K44L, R48K, K155P, K172L, S175I, and K200R;
2) K44L, R48E, K155P, K172L, S175I, and K200R;
3) K44F, R48K, K155P, K172L, S175I, and K200R;
4) K44F, R48E, K155P, K172L, S175I, and K200R;
5) K44L, L46I, K155P, K172L, S175I, and K200R;
6) K44L, L46E, K155P, K172L, S175I, and K200R;
7) K44L, L46F, K155P, K172L, S175I, and K200R;
8) K44L, L46Q, K155P, K172L, S175I, and K200R;
9) K44F, L46I, K155P, K172L, S175I, and K200R;
10) K44F, L46E, K155P, K172L, S175I, and K200R;
11) K44F, L46F, K155P, K172L, S175I, and K200R; and
12) K44F, L46Q, K155P, K172L, S175I, and K200R.

[A5] The granzyme B variant of any one of [A1] to [A4], which comprises one or more amino acid residue mutations relative to human wild-type granzyme B shown in SEQ ID NO: 1.

[A6] The granzyme B variant of any one of [A1] to [A5], wherein protease activity is enhanced more than that of human wild-type granzyme B.

[A7] The granzyme B variant of any one of [A1] to [A6], which has resistance to an inhibitor to human wild-type granzyme B.

[A8] The granzyme B variant of [A7], wherein the inhibitor is PI-9 or heparin.

[A9] An isolated nucleic acid encoding the granzyme B variant of any one of [A1] to [A8].

[A10] A vector comprising the isolated nucleic acid of [A9].

[A1 1] A cell transformed or transduced with the isolated nucleic acid of [A9] or the vector of [A10].

[A12] A cell expressing the granzyme B variant of any one of [A1] to [A8].

[A13] A pharmaceutical composition comprising the isolated nucleic acid of [A9], the vector of [A10], or the cell of [A1 1] or [A12].

[A14] A pharmaceutical composition comprising the granzyme B variant of any one of [A1] to [A8].

[A15] The pharmaceutical composition of any one of [12] to [14], wherein the granzyme B variant is the granzyme B variant of any one of [A1] to [A5].

[A16] The pharmaceutical composition of [A15], which comprises the cell expressing the receptor.

[A17] The pharmaceutical composition of [A16], wherein the receptor and the granzyme B variant are expressed in the same T cell.

[A18] The pharmaceutical composition of [18] or [19], wherein the granzyme B variant is the granzyme B variant of any one of [A1] to [A5].

[A19] The pharmaceutical composition of [A18], which comprises the cell expressing the chimeric receptor.

[A20] The pharmaceutical composition of [A19], wherein the chimeric receptor and the granzyme B variant are expressed in the same T cell.

[A21] The pharmaceutical composition of any one of [A15] to [A20], wherein the receptor is a chimeric antigen receptor.

[B1] The granzyme B variant of [3] or [A6] or the pharmaceutical composition of [12] or [18], wherein *in vitro* protease activity of the granzyme B variant is 1.5 times or more than that of human wild-type granzyme B.

[B2] The granzyme B variant or pharmaceutical composition of [B1], wherein the *in vitro* protease activity of the granzyme B variant is twice or more than that of human wild-type granzyme B.

[B3] The granzyme B variant of [4] or [A7] or the pharmaceutical composition of [12] or [18], wherein *in vitro* protease activity in the presence of the inhibitor is 1.1 times or more, 1.2 times or more, 1.3 times or more, 1.4 times or more, or 1.5 times or more than that of human wild-type granzyme B.

[B4] The granzyme B variant or pharmaceutical composition of [B3], wherein the *in vitro* protease activity in the presence of the inhibitor is 1.1 times or more, 1.2 times or more, 1.3 times or more, 1.4 times or more, 1.5 times or more, or twice or more than that of human wild-type granzyme B.

[B5] The granzyme B variant or pharmaceutical composition of [B3] or [B4], wherein the inhibitor is PI-9 or heparin.

[B6] An isolated nucleic acid encoding the granzyme B variant of any one of [B1] to [B5].

[B7] A vector comprising the isolated nucleic acid of [B6].

[B8] A cell transformed or transduced with the isolated nucleic acid of [B6] or the vector of [B7].

[B9] A cell expressing the granzyme B variant of any one of any one of [B1] to [B5].

[B10] A pharmaceutical composition comprising the isolated nucleic acid of [B6], the vector of [B7], or the cell of [B8] or [B9].

[B11] A pharmaceutical composition comprising the granzyme B variant of any one of [B1] to [B5].

[B12] The pharmaceutical composition of any one of [B1] to [B5],

wherein the receptor is a chimeric receptor which comprises an extracellular binding domain, a transmembrane domain, and an intracellular signaling domain, and binds to the ligand *via* the extracellular binding domain.

[B13] The pharmaceutical composition of any one of [B1] to [B5], wherein the receptor is a T-cell receptor whose ligand is a neoantigen.

[B14] The pharmaceutical composition of any one of [B1] to [B5], [B12], and [B13], wherein the granzyme B variant is the granzyme B variant of any one of [1], [2], and [A1] to [A5].

[B15] The pharmaceutical composition of any one of [B1] to [B5] and [B12] to [B14], which comprises the cell expressing the receptor.

[B16] The pharmaceutical composition of [B15], wherein the receptor and the granzyme B variant are expressed in the same T cell.

[B17] The pharmaceutical composition of any one of [B1] to [B5],

wherein the antigen-binding molecule comprises a linker that is cleavable by a protease, and
wherein the extracellular binding domain is capable of binding to the antigen-binding molecule after cleavage of the linker.

[B18] The pharmaceutical composition of any one of [B1] to [B5], and [B17], wherein the granzyme B variant is the granzyme B variant of any one of [1], [2] and [A1] to [A5].

[B19] The pharmaceutical composition of any one of [B1] to [B5], [B17], and [B18], which comprises the cell expressing the chimeric receptor.

[B20] The pharmaceutical composition of [B19], wherein the chimeric receptor and the granzyme B variant are expressed in the same T cell.

[B21] The pharmaceutical composition of any one of [B12] and [B14] to [B20], wherein the chimeric receptor is a chimeric antigen receptor.

[C1] The pharmaceutical composition of any one of [10] to [23], which is for use in the treatment or prevention of cancer.

[C2] The pharmaceutical composition of any one of [10] to [23], which is for use in the treatment or prevention of an inflammatory disease.

[C3] The granzyme B variant of any one of [1] to [5], [A1] to [A8], and [B1] to [B5] or the cell of [8] or [9], which is for use in the treatment or prevention of cancer or an inflammatory disease.

[C4] A method of treating or preventing cancer or an inflammatory disease, wherein the method comprises administering the granzyme B variant of any one of [1] to [5], [A1] to [A8], and [B1] to [B5] or the cell of [8] or [9].

[C5] The method of [C4], further comprising administering a cell expressing a receptor, wherein the receptor activates the cell expressing the receptor by its binding to a ligand.

[C6] The method of [C5], wherein the receptor is a chimeric receptor that comprises an extracellular binding domain, a transmembrane domain, and an intracellular signaling domain, and binds to the ligand *via* the extracellular binding domain.

[C7] The method of [C6], further comprising administering an antigen-binding molecule, wherein the antigen-binding molecule has an ability to bind to a target antigen, and wherein the chimeric receptor is capable of binding to a cell expressing the target antigen *via* the binding of the extracellular binding domain to the antigen-binding molecule.

[C8] The method of [C7], wherein the antigen-binding molecule comprises a linker that is cleavable by a protease, and wherein the extracellular binding domain is capable of binding to the antigen-binding molecule after cleavage of the linker.

[C9] The method of [C5], wherein the receptor is a T cell receptor whose ligand is a neoantigen.

[C10] The method of any one of [C5] to [C9], wherein the administration is an administration of a T cell expressing the receptor and the granzyme B variant.

[C11] Use of the granzyme B variant of any one of [1] to [5] or the cell of [8] or [9] in the manufacture of a therapeutic agent or preventive agent for cancer or an inflammatory disease.

[D1] A method of producing an isolated nucleic acid encoding the granzyme B variant of any one of [1] to [5], [A1] to [A8], and [B1] to [B5].

[D2] A method of producing a vector comprising the isolated nucleic acid of [6], [A9], or [B6].

[D3] A method of producing a cell transformed or transduced with the isolated nucleic acid of [6], [A9] or [B6], or the vector of [7], [A10] or [B7].

[D4] A method of producing a cell expressing the granzyme B variant of any one of [1] to [5], [A1] to [A8], and [B1] to [B5].

[Effects of the Invention]

In one non-limiting embodiment, the granzyme B variants of the present disclosure have higher protease activity and/or resistance to inhibitors compared to wild-type granzyme B. Pharmaceutical compositions comprising a granzyme B variant having such advantageous effects or a cell expressing the variant of the present disclosure are more advantageous than wild-type granzyme B and the prior art granzyme B variants in inducing cell death of target cells. Furthermore, cell death specific to target cells can be induced by using the granzyme B variants of the present disclosure in combination with pharmaceuticals using cells expressing a receptor and/or a therapeutic antibody.

[Brief Description of Drawings]

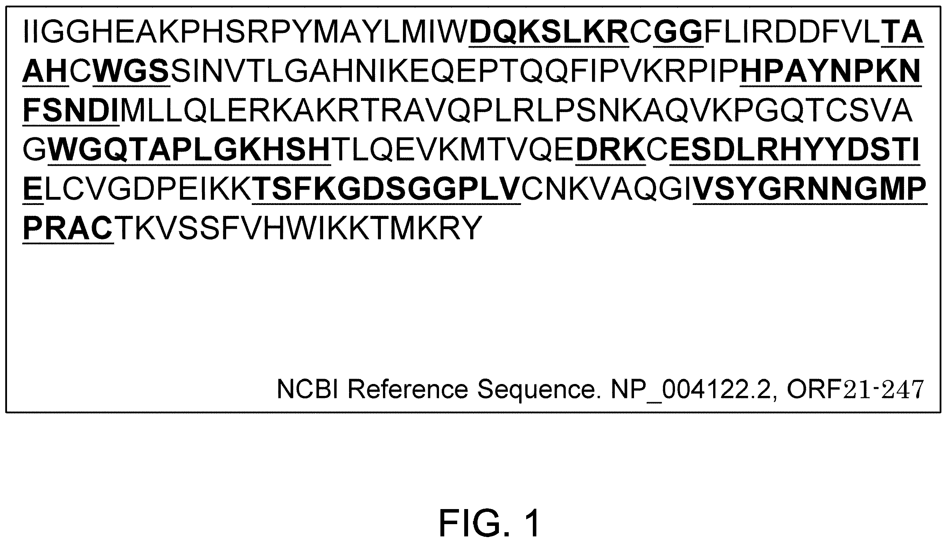

Fig. 1 shows the amino acid sequence (SEQ ID NO: 2) of amino acid numbers 21 to 247 (the amino acids at positions 21 to 247 from the N-terminal side) in the amino acid sequence of human wild-type granzyme B (NCBI Reference Sequence. NP_004122.2; SEQ ID NO: 1). In the sequence, the amino acids at the positions where alterations were made in Example 2 are underlined.

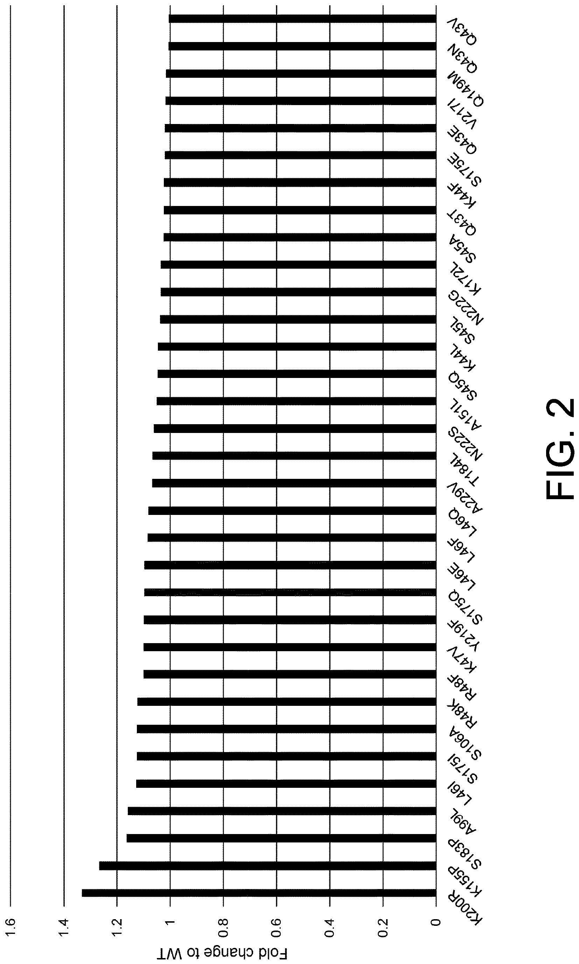

Fig. 2 shows the results of measuring the protease activity of granzyme B variants in Example 4-1. The longitudinal axis indicates the relative value of the protease activity of each granzyme B variant when the protease activity of wild-type granzyme B is set to 1. The horizontal axis indicates the amino acid substitution in the variants measured.

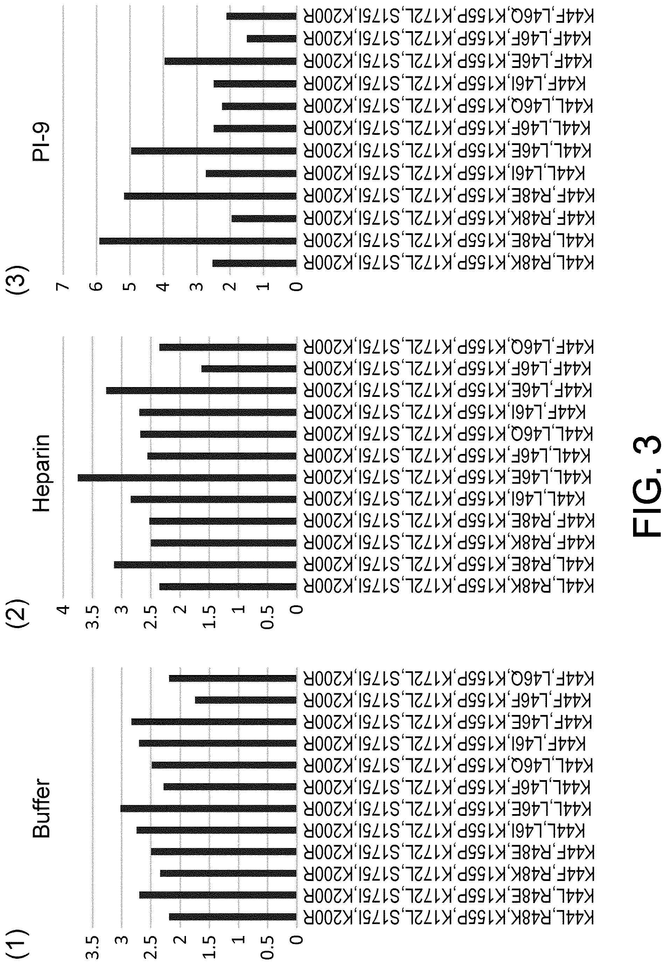

Fig. 3 shows the results of measuring the protease activity of granzyme B variants in Example 4-2. The longitudinal axis indicates the relative value of the protease activity of each granzyme B variant when the protease activity of wild-type granzyme B is set to 1 (1) in the absence of inhibitor (Buffer), (2) in the presence of heparin (Heparin), or (3) in the presence of PI-9 (PI-9). The horizontal axis indicates the amino acid substitutions in the variants measured.

[Description of Embodiments]

I. DEFINITIONS

Unless defined otherwise, technical and scientific terms used herein have the same meaning as commonly understood by one skilled in the art to which the present invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application. All references cited herein, including patent applications and publications, are incorporated herein by reference in their entirety.

For purposes of interpreting the present specification, the following definitions apply and whenever appropriate, terms used in the singular also include the plural and vice versa. It is to be understood that the terminology used herein is for the purpose of describing particular embodiments only, and is not intended to be limiting. In the event that any definition set forth below conflicts with any document incorporated herein by reference, the definition set forth below shall control.

The term "granzyme B," as used herein, refers to any wild-type granzyme B from any vertebrate source, including mammals such as primates (*e.g.,* humans) and rodents *(e.g.,* mice and rats), unless otherwise indicated. The term encompasses "full-length" unprocessed granzyme B as well as any form of granzyme B that results from processing in the cell. The term also encompasses naturally occurring variants of granzyme B, *e.g.,* splice variants or allelic variants. The amino acid sequence of an exemplary human wild-type granzyme B is shown in SEQ ID NO: 1 (NCBI Reference Sequence. NP_004122.2), but is not limited thereto and includes variants of which amino acid sequences are partially different. Such variants include human wild-type granzyme B represented by the amino acid sequence having 90% or more, 95% or more, 97% or more, or 99% or more sequence homology with SEQ ID NO: 1.

As used herein, a "mutation" and an "alteration" are interchangeably used and include an "addition" of an amino acid residue to an amino acid sequence, a "deletion" of an amino acid residue from an amino acid sequence, an "insertion" of an amino acid residue into an amino acid sequence, and/or a "substitution" of an amino acid residue in an amino acid sequence. For obtaining a variant (mutant) with a desired feature (*e.g.*, protease activity or resistance to inhibitors), any combination of an addition, deletion, insertion, and substitution can be introduced. In one embodiment, the granzyme B variants of the present disclosure comprise one or more amino acid residue substitutions.

When the position of each amino acid in granzyme B is indicated herein, the corresponding amino acid number in the amino acid sequence of human wild-type granzyme B exemplified in SEQ ID NO: 1 (in the case of SEQ ID NO: 1, the consecutive number in the amino acid sequence in which the position of the N-terminal amino acid is 1) is designated. For example, when a granzyme B variant of the present disclosure is shown as "comprising the amino acid residue F (Phe) at position 44", it means that the amino acid residue at position 44 from the N-terminal side which constitutes the granzyme B variant is F (Phe).

Moreover, when an amino acid alteration in granzyme B is indicated herein, the amino acid residues before (*i.e.,* wild-type granzyme B) and after the alteration at the position altered are indicated respectively to the left and right, or the left or right, of the amino acid number of the position (*e.g.*, in single-letter notation). For example, when the amino acid residue at the position corresponding to the amino acid residue K (Lys) at position 44 from the N-terminal side in the amino acid sequence shown in SEQ ID NO: 1 is altered to F (Phe), the amino acid alteration is herein represented as K44F. Furthermore, when simply indicating that the amino acid residue at position 44 from the N-terminal side in granzyme B is altered to F (Phe), it is represented as 44F.

Even if the amino acid sequence of granzyme B to be altered has part different from the sequence shown in SEQ ID NO: 1, those skilled in the art can appropriately determine where the positions of alterations shown herein actually correspond to in granzyme B to be altered (*e.g.*, by performing sequence alignment).

In the present disclosure, the granzyme B variant comprises one or more amino acid residues selected from 1) to 20) below:

1) T, E, N or V at position 43; 2) L or F at position 44; 3) Q, L or A at position 45; 4) I, E, F or Q at position 46; 5) V at position 47; 6) F or K at position 48; 7) L at position 99; 8) A at position 106; 9) M at position 149; 10) L at position 151; 11) P at position 155; 12) L at position 172; 13) Q, E or I at position 175; 14) P at position 183; 15) L at position 184; 16) R at position 200; 17) I at position 217; 18) F at position 219; 19) G or S at position 222; and 20) V at position 229.

In another aspect, the granzyme B variant of the present disclosure comprises any one of the combination of amino acid residues of 1) to 12) below:

1) L at position 44, K at position 48, P at position 155, L at position 172, I at position 175, and R at position 200;
2) L at position 44, E at position 48, P at position 155, L at position 172, I at position 175, and R at position 200;
3) F at position 44, K at position 48, P at position 155, L at position 172, I at position 175, and R at position 200;
4) F at position 44, E at position 48, P at position 155, L at position 172, I at position 175, and R at position 200;
5) L at position 44, I at position 46, P at position 155, L at position 172, I at position 175, and R at position 200;
6) L at position 44, E at position 46, P at position 155, L at position 172, I at position 175, and R at position 200;
7) L at position 44, F at position 46, P at position 155, L at position 172, I at position 175, and R at position 200;
8) L at position 44, Q at position 46, P at position 155, L at position 172, I at position 175, and R at position 200;
9) F at position 44, I at position 46, P at position 155, L at position 172, I at position 175, and R at position 200;
10) F at position 44, E at position 46, P at position 155, L at position 172, I at position 175, and R at position 200;
11) F at position 44, F at position 46, P at position 155, L at position 172, I at position 175, and R at position 200;
12) F at position 44, Q at position 46, P at position 155, L at position 172, I at position 175, and R at position 200.

"Protease activity" herein refers to, especially in the context of granzyme B, the activity of granzyme B to cleave its substrate. Methods of evaluating the protease activity of granzyme B are well known to those skilled in the art, and various activity measurement kits and synthetic substrates are commercially available. As examples of such synthetic substrates, synthetic peptides having a recognition sequence of granzyme B (*e.g.*, Ile-Glu-Pro-Asp (IEPD)) which are labeled with a detectable substance (*e.g.,* p-nitroanilide (pNA)) are known (*e.g.,* Ac-IEPD-pNA). Cleavage of a synthetic substrate by granzyme B releases a free detectable substance, which can be quantitatively determined with a fluorometer or a spectrophotometer. As an example, evaluation of the protease activity of granzyme B variants can be performed by the method described in Example 4 of the present disclosure. For example, evaluation of the protease activity in the presence of inhibitors can be performed using the evaluation system described in Example 4 and under the same concentration conditions of granzyme B and inhibitors as in the Example.

The protease activity of the granzyme B variants of the present disclosure is preferably enhanced compared to that of wild-type granzyme B, and is, for example, preferably 1.1 times or more, 1.2 times or more, 1.3 times or more, 1.4 times or more, or 1.5 times or more higher, more preferably twice or more or 2.5 times or more higher, and particularly preferably 3 times or more higher than the protease activity of wild-type granzyme B.

The protease activity of wild-type granzyme B is known to be inhibited by "inhibitors" such as PI-9 and heparin. The granzyme B variants of the present disclosure preferably have resistance to such inhibitors. Herein, "resistance to an inhibitor" refers to an ability to exert protease activity higher than that of wild-type granzyme B in the presence of such an inhibitor. The protease activity of the granzyme B variants of the present disclosure in the presence of such an inhibitor is preferably 1.1 times or more, 1.2 times or more, 1.3 times or more, 1.4 times or more, or 1.5 times or more higher, more preferably twice or more, 2.5 times or more, 3 times or more, or 3.5 times or more higher, and particularly preferably 4 times or more, 4.5 times or more, 5 times or more, or 5.5 times or more higher than that of wild-type granzyme B.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," or "transductant" and "transduced cells," which include the primary transformed cell or transduced cell and progeny derived therefrom without regard to the number of passages. Progeny may not be completely identical in nucleic acid content to a parent cell, but may contain mutations. Mutant progeny that have the same function or biological activity as screened or selected for in the originally transformed cell or transduced cell are included herein.

Herein, unless otherwise indicated, a "cell expressing granzyme B" may be a cell expressing endogenous granzyme B or a cell expressing granzyme B by gene transfer. As cells expressing endogenous granzyme B, for example, cytotoxic lymphocytes - T cells and NK cells - are known. On the other hand, cells that are transfected to express granzyme B are not limited to T cells and NK cells, and recombinant granzyme B can be obtained by expressing recombinant granzyme B in various cells (for example, by purifying granzyme B from their culture supernatant). Furthermore, it is possible to introduce a gene expressing granzyme B into cells (*e.g.,* peripheral blood mononuclear cells (PBMCs)) derived from an individual (*e.g.,* a healthy donor or a patient suffering from a particular disease), and to administer the transgenic cells to the same or different individual. Transgenic cells expressing granzyme B may be treated to differentiate into a particular cell type (*e.g.,* cytotoxic T cells) and then administered to an individual. As a method of introducing a gene encoding granzyme B into cells, various gene transfer techniques well known to those skilled in the art can be used.

Cells expressing granzyme B may be administered in combination with cells expressing a chimeric receptor. Moreover, cells expressing both granzyme B and a chimeric receptor may be used, and such cells can be produced by simultaneous or separate gene transfer of granzyme B and a chimeric receptor.

When a granzyme B variant is expressed in a cell expressing endogenous granzyme B, such as a T cell or NK cell, the endogenous wild-type granzyme B in the cell may or may not be knocked out.

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation or composition would be administered. An "active ingredient" can be constituted as an antibody, polypeptide or the like, or as a cell expressing an antibody, polypeptide or the like (*e.g.,* a granzyme B variant of the present disclosure). For example, when a cell transformed or transduced with a nucleic acid encoding a granzyme B variant of the present disclosure or a vector comprising such a nucleic acid is administered to a patient for purposes of treatment or prevention, a preparation comprising such a cell can be referred to as a "pharmaceutical formulation" or "pharmaceutical composition".

A "pharmaceutically acceptable carrier" refers to an ingredient in a pharmaceutical formulation or pharmaceutical composition, other than an active ingredient, which is nontoxic to a subject. A pharmaceutically acceptable carrier includes, but is not limited to, a buffer, excipient, stabilizer, or preservative.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

In one aspect, the present disclosure provides granzyme B variants, cells expressing a granzyme B variant, or pharmaceutical compositions comprising them.

In one embodiment, the pharmaceutical compositions of the present disclosure can be used in combination with a cell expressing a receptor. The receptor is one that activates a cell expressing the receptor by its binding to a ligand, and includes, for example, a chimeric receptor that binds to a ligand *via* the extracellular binding domain and a T-cell receptor whose ligand is a neoantigen, but is not limited thereto. The pharmaceutical compositions of the present disclosure may comprise a cell expressing a receptor and a cell expressing a granzyme B variant, and the receptor and the granzyme B variant may be expressed in the same cell (*e.g.,* a T cell).

Such pharmaceutical compositions of the present disclosure can be used in combination with an antigen-binding molecule that is capable of binding to the extracellular binding domain of a chimeric receptor. The antigen-binding molecule has an ability to bind to a target antigen, and the chimeric receptor can bind to a cell expressing the target antigen *via* the binding of its extracellular binding domain to the antigen-binding molecule. In a certain embodiment, the antigen-binding molecule comprises a linker that is cleavable by a protease, and the extracellular binding domain can bind to the antigen-binding molecule after cleavage of the linker. The pharmaceutical compositions of the present disclosure may comprise a cell expressing a chimeric receptor and a cell expressing a granzyme B variant, and an antigen-binding molecule capable of binding to the extracellular binding domain of the chimeric receptor. The receptor and the granzyme B variant may be expressed in the same cell (*e.g.,* a T cell).

When one or more of a granzyme B variant, a cell expressing a granzyme B variant, a cell expressing a receptor, a cell expressing a receptor and a granzyme B variant, and an antigen-binding molecule capable of binding to the extracellular binding domain of a chimeric receptor are used in combination, they can be used (*e.g.,* administered to an individual) simultaneously, separately, or sequentially.

In one embodiment, the pharmaceutical compositions of the present disclosure are for use in damaging cells, inducing cell death, suppressing cell proliferation, or treating or preventing cancer or inflammatory diseases.

The term "chimeric receptor" refers to a recombinant polypeptide comprising at least an extracellular binding domain, a transmembrane domain, and an intracellular signaling domain, which polypeptide provides specificity for a target cell, *e.g.,* a cancer cell, and produces intracellular signals when expressed in an immune effector cell. The term "chimeric antigen receptor" or "CAR" means a chimeric receptor whose extracellular binding domain binds to an antigen.

The term "extracellular binding domain" means any proteinaceous molecule or part thereof that can specifically bind to a given molecule such as an antigen, and includes, for example, a single-chain antibody (scFv) in which a light chain (VL) and a heavy chain (VH) of a monoclonal antibody variable region specific to a tumor antigen or the like are connected in tandem. An extracellular binding domain can be rephrased as an extracellular recognition domain.

The term "transmembrane domain" includes a polypeptide that is located between the extracellular binding domain and the intracellular signaling domain and has the function of penetrating the cell membrane.

The term "intracellular signaling domain" means any oligopeptide domain or polypeptide domain that is known to function to transmit a signal that causes activation or inhibition of a biological process within a cell, *e.g.*, activation of an immune cell such as a T cell or NK cell, and the domain comprises at least one stimulatory molecule signaling domain derived from a T-cell stimulatory molecule and at least one costimulatory molecule signaling domain derived from a T-cell costimulatory molecule.

The term "T-cell receptor whose ligand is a neoantigen" herein includes T-cell receptors designed to recognize neoantigens, which are mutant antigens resulting from genetic mutations in cancer cells.

Herein, the term "antigen-binding molecule" refers, in its broadest sense, to a molecule that specifically binds to an antigenic determinant (epitope). In one embodiment, the antigen-binding molecule is an antibody, antibody fragment, or antibody derivative. In one embodiment, the antigen-binding molecule is a non-antibody protein, or a fragment thereof, or a derivative thereof.

Herein, "antigen-binding domain" refers to a region that specifically binds and is complementary to the whole or a portion of an antigen. Herein, an antigen-binding molecule comprises an antigen-binding domain. When the molecular weight of an antigen is large, an antigen-binding domain can only bind to a particular portion of the antigen. The particular portion is called "epitope". In one embodiment, an antigen-binding domain comprises an antibody fragment which binds to a particular antigen. An antigen-binding domain can be provided from one or more antibody variable domains. In a non-limiting embodiment, the antigen-binding domains comprise both the antibody light chain variable region (VL) and antibody heavy chain variable region (VH). Examples of such antigen-binding domains include "single-chain Fv (scFv)", "single-chain antibody", "Fv", "single-chain Fv2 (scFv2)", "Fab", and "Fab‴". In other embodiments, an antigen-binding domain comprises a non-antibody protein which binds to a particular antigen, or a fragment thereof. In a specific embodiment, an antigen-binding domain comprises a hinge region.

Herein, "specifically binds" means binding in a state where one of the molecules involved in specific binding does not show any significant binding to molecules other than a single or a number of binding partner molecules. Furthermore, it is also used when an antigen-binding domain is specific to a particular epitope among multiple epitopes contained in an antigen. When an epitope bound by an antigen-binding domain is contained in multiple different antigens, antigen-binding molecules comprising the antigen-binding domain can bind to various antigens that have the epitope.

The term "antibody" herein is used in the broadest sense and encompasses various antibody structures, including but not limited to monoclonal antibodies, polyclonal antibodies, multispecific antibodies (*e.g.*, bispecific antibodies), and antibody fragments so long as they exhibit the desired antigen-binding activity.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, *e.g.,* Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, *e.g.,* Portolano et al., J. Immunol. 150:880-887 (1993); Clarkson et al., Nature 352:624-628 (1991).

In several embodiments, the present disclosure provides methods of damaging cells, inducing cell death, suppressing cell proliferation, treating inflammatory diseases, treating cancer, or preventing cancer, the method comprising administering an effective amount of a granzyme B variant, a cell expressing the granzyme B variant, or a pharmaceutical composition comprising them of the present disclosure (hereinafter may be collectively referred to as "pharmaceutical composition and such of the present disclosure"). In some embodiments, the "effective amount" in the present invention means a dose of the pharmaceutical composition and such of the present disclosure that is effective in damaging cells, inducing cell death, suppressing cell proliferation, treating inflammatory diseases, treating cancer, or preventing cancer in individuals.

In several embodiments, "treatment/treating/therapeutic" according to the present invention means that a pharmaceutical composition and such of the present disclosure decreases the number of cancer cells in individuals, suppresses cancer cell proliferation, decreases tumor size (volume and/or weight), suppresses tumor enlargement, suppresses infiltration of cancer cells into peripheral organs, suppresses cancer cell metastasis, or ameliorates various symptoms caused by cancer. Furthermore, in several embodiments, "prevention/preventing/prophylactic" according to the present invention refers to inhibiting increase in the number of cancer cells due to repopulation of cancer cells that have decreased, inhibiting repopulation of cancer cells whose proliferation have been suppressed, and inhibiting the decreased tumor size (volume and/or weight) to become large again.

[Example of Application]

The granzyme B variants of the present disclosure can be used in combination with administration of a cell expressing a receptor. An example of the receptor includes a chimeric receptor comprising an extracellular binding domain, a transmembrane domain, and an intracellular signaling domain. Furthermore, another example includes a T-cell receptor designed to recognize a neoantigen that cannot be recognized by normal T cells. As an embodiment of combinations of techniques utilizing these receptors and the granzyme B variants of the present disclosure, there is a means of collecting T cells from a patient, introducing a gene encoding a chimeric receptor (*e.g.*, a chimeric antigen receptor) and a gene encoding a granzyme B variant into the T cells, and transferring them back into the patient. (Without being limited to that, it is also possible to separately introduce the genes and transfer the T cells into the patient.) The chimeric antigen receptor recognizes a surface antigen of a cell such as a cancer cell to activate the T cells, and enhanced cytotoxic activity is exerted by a granzyme B variant of the present disclosure, and thereby a high therapeutic effect is expected.

Similarly, by introducing a gene encoding a T-cell receptor whose ligand is a neoantigen and a gene encoding a granzyme B variant into T cells collected from a patient, and then transferring them back into the patient, a high therapeutic effect from the combination is expected.

Moreover, in combinations of the granzyme B variants of the present disclosure and chimeric receptors, combinations further combined with antigen-binding molecules (such as antibodies) can also be used. In this case, an antigen-binding molecule has an ability to bind to a target antigen, and a chimeric receptor can bind to cells expressing the target antigen *via* the binding of the extracellular binding domain to the antigen-binding molecule. As an embodiment of the antigen-binding molecule, the antigen-binding molecule is prepared in advance to comprise a linker that is cleavable by a protease, and the chimeric receptor can be designed to be capable of binding to the antigen-binding molecule after cleavage of the linker. As an embodiment of such combinations, there is a means of collecting T cells from a patient, introducing a gene encoding a chimeric receptor and a gene encoding a granzyme B variant into the T cells, transferring them back into the patient, and separately administering a pharmaceutical composition comprising an antigen-binding molecule to the patient.

[Examples]

Herein below, Examples of construction, expression, purification, gene transfer, and use for treating injuries and diseases of variant granzymes are described, but methods of performing this patent are not limited to these Examples.

[Example 1] Construction of a vector expressing human wild-type granzyme B

The sequence of human wild-type granzyme B (NCBI Reference Sequence. NP_004122.2) was genetically synthesized. The nucleotide sequences respectively encoding an artificial secretion signal sequence (MGILPSPGMPALLSLVSLLSVLLMGCVAETG (SEQ ID NO: 3)) and an enterokinase recognition sequence (DDDDK (SEQ ID NO: 4)) were fused to the 5'-end of the nucleotide sequence encoding amino acid numbers 21-247 (SEQ ID NO: 2) of the ORF encoding granzyme B, and the nucleotide sequence encoding a histidine tag was added to the C-terminal side (J Vis Exp. 2015 Jun 10; (100): e52911). The nucleotide sequence encoding that sequence (SEQ ID NO: 5) was inserted into a mammalian cell expression vector.

Furthermore, when an amino acid alteration position in granzyme B is indicated herein, the corresponding amino acid number in the amino acid sequence of human wild-type granzyme B (NCBI Reference Sequence. NP_004122.2) shown in SEQ ID NO: 1 is designated.

[Example 2] Production of the nucleotide sequences encoding granzyme B variants

Substitutions of a single amino acid residue with respect to human wild-type granzyme B and substitutions of multiple amino acid residues combining them were performed using PCR reactions by a method known to those skilled in the art.

The positions of amino acids to be altered were selected by identifying amino acid residues close to the substrate-binding site based on the crystal structure of human wild-type granzyme B (the underlined amino acids in Fig. 1). For these amino acid residues, primers were designed that encode substitution product in which the amino acid residue was substituted with any of all the 18 amino acids except the amino acid before the alteration and cysteine. By methods known to those skilled in the art such as PCR using these primers, a total of 1476 nucleotide sequences were produced that encoded granzyme B variants in which a single amino acid has been substituted.

Moreover, by similar methods, nucleotide sequences encoding granzyme B variants with multiple amino acid substitutions were produced.

[Example 3] Expression and purification of granzyme B variants

The nucleotide sequences encoding granzyme B produced in Example 2 were transfected into 1 mL of Expi293 (Invitrogen) culture medium by the method specified by the supplier. After four days, the culture supernatant was collected, and enterokinase was added at a final concentration of 0.3 μg/mL and reacted at 4°C for 16 hours. One-tenth volume of a binding buffer (250 mM Tris, 3M NaCl, pH 7.5) was added to the reaction solution, and then 50 μL of Ni Sepharose Excel (GE healthcare, 17371201) suspended in an equilibrium buffer (25 mM Tris-HCl, 500 mM NaCl, pH 7.5) was added. After reaction at 4°C for one hour, the reaction solution was added to a filter plate (Merck, MSGVS2210). The granzyme B-bound Ni Sepharose Excel was washed five times with 200 μL of the equilibrium buffer, and then reacted with an elution buffer (25 mM Tris-HCl, 500 mM NaCl, 500 mM Imidazole, pH 7.5) at 4°C for 15 minutes to elute granzyme B. The concentration of the purified granzyme B was calculated using the absorbance at 280 nm and the extinction coefficient calculated by the PACE method of the eluted granzyme B solution (Protein Science (1995) 4, 2411-2423).

[Example 4] Measurement of the protease activity of granzyme B variants (4-1) Granzyme B variants (a single amino acid substitution)

The expressed and purified granzyme B was suspended in a twice diluted enzyme reaction Buffer (2x Reaction buffer, Promokine, PK-CA577-1068-80) and mixed with a reaction substrate, Ac-IEPD-pNA, at a final concentration of 0.5 mM (Enzo, BML-P133). The absorbance at 405 nm of the reaction solution was measured. Fig. 2 shows the relative protease activity of the granzyme B variants measured relative to wild-type granzyme B.

The granzyme B activity shown in Fig. 2 is defined by the following formula.

$$\text{Fold change} = (\text{the protease activity of a granzyme B variant}) / (\text{the protease activity of wild-type granzyme B})$$

Furthermore, the protease activity of granzyme B is defined by the following formula.

$$\text{Protease activity} = (\text{the change in absorbance at 405 nm of the reaction solution in unit time}) / (\text{unit time})$$

(4-2) Granzyme B variants (multiple amino acid substitutions)

As a result of measuring 1476 variants in the above-mentioned comprehensive measurement, 33 variants were found to have increased activity relative to wild-type granzyme B, and 1443 variants had lower activity than the wild type. With respect to these 33 alterations that increase the protease activity of granzyme B, multiple variants of granzyme B variants in which multiple mutations had been combined were produced by the methods described in Example 2. Of the variants produced, examples in which the increase in activity was particularly remarkable are shown in Table 1. In Table 1, the positions of amino acid substitutions in the variants refer to the corresponding amino acid numbers in the amino acid sequence of human wild-type granzyme B shown in SEQ ID NO: 1, and the amino acid sequences of the variants indicate the variant amino acid sequences corresponding to amino acid numbers 21-247 of human wild-type granzyme B shown in SEQ ID NO: 1.

[Table 1]

| Amino acid substitutions in variants | Amino acid sequence of variants |
|---|---|
| K44L, R48K, K155P, K172L, S175I, K200R | SEQ ID NO: 6 |
| K44L, R48E, K155P, K172L, S175I, K200R | SEQ ID NO: 7 |
| K44F, R48K, K155P, K172L, S175I, K200R | SEQ ID NO: 8 |
| K44F, R48E, K155P, K172L, S175I, K200R | SEQ ID NO: 9 |
| K44L, L461, K155P, K172L, S175I, K200R | SEQ ID NO: 10 |
| K44L, L46E, K155P, K172L, S175I, K200R | SEQ ID NO: 11 |
| K44L, L46F, K155P, K172L, S175I, K200R | SEQ ID NO: 12 |
| K44L, L46Q, K155P, K172L, S175I, K200R | SEQ ID NO: 13 |
| K44F, L461, K155P, K172L, S175I, K200R | SEQ ID NO: 14 |
| K44F, L46E, K155P, K172L, S175I, K200R | SEQ ID NO: 15 |
| K44F, L46F, K155P, K172L, S175I, K200R | SEQ ID NO: 16 |
| K44F, L46Q, K155P, K172L, S175I, K200R | SEQ ID NO: 17 |

When the protease activity was measured in the presence of an inhibitor, wild-type granzyme B or the granzyme B variants were incubated at 37°C for one hour in the presence of 40 $\mu$M heparin (Heparin sodium salt from porcine intestinal mucosa, Sigma-Aldrich, H3393-50KU) or 0.5 $\mu$M PI-9 (Recombinant Human Serpin B9/SERPINB9 (C-6His) (Novoprotein, CJ32)) and 0.5 mM DTT, and then mixed with 1 mM Ac-IEPD-pNA solution at a volume ratio of 1:1. The twice diluted enzyme reaction Buffer (2x Reaction buffer, Promokine, PK-CA577-1068-80) was used to prepare all solutions. In measuring the protease activity of the produced variants in the absence of an inhibitor, they were incubated with the enzyme reaction Buffer containing 0.5 mM DTT instead of the inhibitor suspension at 37°C for one hour, and then mixed with 1 mM Ac-IEPD-pNA solution at a volume ratio of 1:1 and measured. Measurement of the absorbance was performed in the same manner as described in Example 4-1. The results of the measurement are shown in Fig. 3-(1) to -(3). As a result of the measurement, it was confirmed that the granzyme B activity was increased compared to wild-type granzyme B and that the variants maintained high protease activity even in the presence of inhibitors.

[Example 5] Expression of granzyme B variants in a NK cell line

A secretion signal sequence derived from wild-type granzyme B and a cathepsin C/H recognition sequence are fused to the N-terminal side of the granzyme B variants produced in Example 4 and the amino acid sequence corresponding to amino acid numbers 21-247 of wild-type granzyme B (SEQ ID NO: 1), and a FLAG tag is fused to their C-terminal side. The nucleotide sequences encoding these fusions are transfected into an NK cell line (NKL, ATCC No. ) by a mammalian expression vector, pGL4.30 (Promega, E8481). This NK cell line is selected with hygromycin B. The cell lines of granzyme B variants are membrane-permeabilized using eBioscience™ Foxp3/Transcription Factor Staining Buffer Set (Invitrogen, 00-5523-00) by the method specified by one skilled in the art, and are fluorescently stained utilizing an anti-FLAG antibody that recognizes the tag fused to the C-terminus of granzyme B and its isotype control antibody (Biolegend, 637310 and 400508). The cells stained are detected with FACS verse (BD). As a result, a peak associated with granzyme B expression is observed in the cells into which granzyme B is transfected, and it is confirmed that the transfected granzyme B is expressed.

[Example 6] Measurement of the *in vitro* cytotoxic activity of cell lines expressing granzyme B variants

The cell lines expressing granzyme B variants established and a target cell (any one of BxPC-3, HuCCT-1, and MCAS) are added to a 96-well plate, and the anti-EGFR antibody CetuH0-Hl076/CetuL4-k0//CetuH0-Kn125/CetuL4-k0, diluted to each concentration, is added. After reaction at 37°C, LDH release associated with cell death is quantitatively determined using Pierce LDH Cytotoxicity Assay Kit (Thermofisher Scientific, 88954) by the method specified by the supplier. As a result, it is shown that the transgenic cells expressing granzyme B variants exhibit stronger cytotoxic activity than the transgenic cell expressing wild-type granzyme B.

[Example 7] Production of viral vectors encoding granzyme B variants

Retroviral vectors encoding human wild-type granzyme B and granzyme B variants are prepared utilizing pMCs-IRES-GFP Retroviral Vector (Cell Biolabs, Inc., RTV-040) by the method specified by the supplier.

[Example 8] Gene transfer of granzyme B variants to primary T cells

HLA-A2+ peripheral blood mononuclear cells (PBMCs) derived from healthy donors (Biological Specialty Corp., Colmar, PA, USA) are isolated by Ficoll-Paque (GE Healthcare, Piscataway, NJ, USA) density gradient centrifugation. The PBMCs are cultured in a 24-well tissue culture plate in AIM V medium (GIBCO brand; Invitrogen) supplemented with 5% human AB serum (Sigma-Aldrich), 1% MEM non-essential amino acids, 1% penicillin-streptomycin, and 100 U/mL recombinant human IL-2 (BioLegend, San Diego, CA, USA) at $3 \times 10^6$ cells/well, and are activated with 50 ng/mL OKT3 (eBioscience, San Diego, CA, USA). After two days, the cells are collected for retroviral transduction. For transduction, a 24-well non-tissue culture treated plate (BD Biosciences, Franklin Lakes, NJ, USA) is coated using 10 μg/mL recombinant human fibronectin fragment (RetroNectin; Takara Bio Inc., Otsu, Shiga, Japan) at 0.5 mL/well at 4°C overnight. After the incubation, the wells are blocked using 1 mL of Hanks' solution (GIBCO brand; Invitrogen) supplemented with 2.5% human AB serum at room temperature for 30 minutes, and washed with Hanks' solution supplemented with 2.5% N-2-hydroxyethylpiperazine-N'-2-ethanesulfonic acid (HEPES) (GIBCO brand; Invitrogen).

Transduction is performed by a method previously described (Johnson et al., Blood 114, 535-546 (2009)). Briefly, about 2.5 mL of the retroviral supernatant is added to each coated well and then centrifuged at 32°C for two hours at 2000 g. One point five milliliter of the viral supernatant is removed and $1 \times 10^6$ (0.5 mL) of the activated PBMCs are added to each well in the presence of 100 U/mL IL-2. The plate is centrifuged at 1000 g for 10 minutes and then incubated at 37°C overnight.

After the transduction, the cells are washed and maintained in the presence of IL-2 (100 U/mL), and used for experiments five days after the transduction. Expression of granzyme B in the transduced human T cells is determined by flow cytometry after staining with an antibody recognizing the FLAG tag labeled at the C-terminus of granzyme B (Biolegend, 637310) or its isotype control antibody (Biolegend, 400508).

[Example 9] Evaluation of the cytotoxic activity of T cells transfected with granzyme B variants

The T cells expressing granzymes prepared in Example 8 and a target cell (any one of BxPC-3, HuCCT-1, and MCAS) are added to a 96-well plate, respectively, and the anti-EGFR anti-CD3 bispecific antibody CetuH0-F760nN17/CetuL4-k0//TR01H113-F760nP17/L0011-k0, diluted to each concentration, is added. After reaction at 37°C, LDH release associated with cell death is quantitatively determined using Pierce LDH Cytotoxicity Assay Kit (Thermofisher scientific, 88954) by the method specified by the supplier. As a result, it becomes clear that the transgenic cells expressing granzyme B variants exhibit stronger cytotoxic activity than the transgenic cell expressing wild-type granzyme B.

[Example 10] Evaluation of the in vitro cytotoxic activity of T cells transfected with granzyme B variants

The cytotoxic activity of the granzyme-transfected T cells produced in Example 8 is also evaluated with BD FACSVerse™ (BD Biosciences). BxPC-3, HuCCT-1, or MCAS, which is a cancer cell, is prepared as a target cell. The target cells are seeded in a 6-well plate at $1 \times 10^5$ cells or $3 \times 10^5$ cells, respectively. The T cells expressing granzyme B variants or wild-type granzyme B are used as effector cells, and are mixed so that the ratio of effector cells to target cells (E:T) is 1:1 or 1:3. Next, the anti-EGFR anti-CD3 bispecific antibody CetuH0-F760nN17/CetuL4-k0//TR01H113-F760nP17/L0011-k0 is added at 10 μg per well. After 48 hours of the addition, the granzyme B-transfected T cells and target cells are collected. Dead cells in the cells collected are stained using Zombie Aqua™ Fixable Viability Kit (BioLegend, 423102), and the granzyme B-expressing T cells are stained using an anti-human CD45 antibody (BioLegend, 304039).

Cytotoxic activity is evaluated by the ratio of residual cancer cells. The ratio of residual cancer cells is calculated as the ratio of CD45-fraction cells in viable cells. These results show enhanced cytotoxic activity of the granzyme-expressing T cells in vitro.

[Industrial Applicability]

The granzyme B variants of the present disclosure exhibit higher protease activity than wild-type granzyme B, and thus are more useful than wild-type granzyme B in treatments that induce cell death of target cells (e.g., cancer cells). Furthermore, the granzyme B variants of the present disclosure exhibit high protease activity even in the presence of inhibitors to wild-type granzyme B, and are therefore more useful than wild-type granzyme B in treatments that induce cell death in tumors that highly express such inhibitors.

**Claims**

1. A granzyme B variant comprising one or more amino acid residues selected from 1) to 20) below:

    1) T, E, N, or V at position 43; 2) L or F at position 44; 3) Q, L, or A at position 45; 4) I, E, F, or Q at position 46; 5) V at position 47; 6) F or K at position 48; 7) L at position 99; 8) A at position 106; 9) M at position 149; 10) L at position 151; 11) P at position 155; 12) L at position 172; 13) Q, E, or I at position 175; 14) P at position 183; 15) L at position 184; 16) R at position 200; 17) I at position 217; 18) F at position 219; 19) G or S at position 222; and 20) V at position 229.

2. The granzyme B variant of claim 1, wherein the variant comprises any one of the combinations of amino acid residues 1) to 12) below:

    1) L at position 44, K at position 48, P at position 155, L at position 172, I at position 175, and R at position 200;
    2) L at position 44, E at position 48, P at position 155, L at position 172, I at position 175, and R at position 200;
    3) F at position 44, K at position 48, P at position 155, L at position 172, I at position 175, and R at position 200;
    4) F at position 44, E at position 48, P at position 155, L at position 172, I at position 175, and R at position 200;
    5) L at position 44, I at position 46, P at position 155, L at position 172, I at position 175, and R at position 200;
    6) L at position 44, E at position 46, P at position 155, L at position 172, I at position 175, and R at position 200;
    7) L at position 44, F at position 46, P at position 155, L at position 172, I at position 175, and R at position 200;
    8) L at position 44, Q at position 46, P at position 155, L at position 172, I at position 175, and R at position 200;
    9) F at position 44, I at position 46, P at position 155, L at position 172, I at position 175 and R at position 200;
    10) F at position 44, E at position 46, P at position 155, L at position 172, I at position 175, and R at position 200;
    11) F at position 44, F at position 46, P at position 155, L at position 172, I at position 175, and R at position 200; and
    12) F at position 44, Q at position 46, P at position 155, L at position 172, I at position 175, and R at position 200.

3. The granzyme B variant of claim 1 or 2, wherein protease activity is enhanced more than that of human wild-type granzyme B.

4. The granzyme B variant of any one of claims 1 to 3, which has resistance to an inhibitor to human wild-type granzyme B.

5. The granzyme B variant of claim 4, wherein the inhibitor is PI-9 or heparin.

6. An isolated nucleic acid encoding the granzyme B variant of any one of claims 1 to 5.

7. A vector comprising the isolated nucleic acid of claim 6.

8. A cell transformed or transduced with the isolated nucleic acid of claim 6 or the vector of claim 7.

9. A cell expressing the granzyme B variant of any one of claim 1 to 5.

10. A pharmaceutical composition comprising the isolated nucleic acid of claim 6, the vector of claim 7, or the cell of claim 8 or 9.

11. A pharmaceutical composition comprising the granzyme B variant of any one of claim 1 to 5.

12. A pharmaceutical composition for use in combination with administration of a cell expressing a receptor, wherein the composition comprises a granzyme B variant or a cell expressing a granzyme B variant,

    wherein the receptor activates the cell expressing the receptor by its binding to a ligand, and
    wherein the granzyme B variant has enhanced protease activity more than that of human wild-type granzyme B and has resistance to an inhibitor to human wild-type granzyme B.

13. The pharmaceutical composition of claim 12, wherein the receptor is a chimeric receptor which comprises an extracellular binding domain, a transmembrane domain, and an intracellular signaling domain, and binds to the ligand *via* the extracellular binding domain.

**14.** The pharmaceutical composition of claim 12, wherein the receptor is a T-cell receptor whose ligand is a neoantigen.

**15.** The pharmaceutical composition of any one of claims 12 to 14, wherein the granzyme B variant is the granzyme B variant of claim 1, 2, or 5.

**16.** The pharmaceutical composition of any one of claim 12 to 15, which comprises the cell expressing the receptor.

**17.** The pharmaceutical composition of claim 16, wherein the receptor and the granzyme B variant are expressed in the same T cell.

**18.** A pharmaceutical composition for use in combination with administration of an antigen-binding molecule and administration of a cell expressing a chimeric receptor, wherein the composition comprises a granzyme B variant or a cell expressing a granzyme B variant,

wherein the antigen-binding molecule has an ability to bind to a target antigen,
wherein the chimeric receptor comprises an extracellular binding domain, a transmembrane domain, and an intracellular signaling domain and is capable of binding to a cell expressing the target antigen *via* the binding of the extracellular binding domain to the antigen-binding molecule, and
wherein the granzyme B variant has enhanced protease activity more than that of human wild-type granzyme B and has resistance to an inhibitor to human wild-type granzyme B.

**19.** The pharmaceutical composition of claim 18,

wherein the antigen-binding molecule comprises a linker that is cleavable by a protease, and
wherein the extracellular binding domain is capable of binding to the antigen-binding molecule after cleavage of the linker.

**20.** The pharmaceutical composition of claim 18 or 19, wherein the granzyme B variant is the granzyme B variant of claim 1, 2, or 5.

**21.** The pharmaceutical composition of any one of claim 18 to 20, which comprises the cell expressing the chimeric receptor.

**22.** The pharmaceutical composition of claim 21, wherein the chimeric receptor and the granzyme B variant are expressed in the same T cell.

**23.** The pharmaceutical composition of any one of claim 13 and 15 to 22, wherein the chimeric receptor is a chimeric antigen receptor.

IIGGHEAKPHSRPYMAYLMIW**DQKSLKR**CGG FLIRDDFVL**TA**

**AH**C**WGS**SINVTLGAHNIKEQEPTQQFIPVKRPIP**HPAYNPKN**

**FSND**IMLLQLERKAKRTRAVQPLRLPSNKAQVKPGQTCSVA

G**WGQTAPLGKHSH**TLQEVKMTVQE**DRK**C**ESDLRHYYDSTI**

**E**LCVGDPEIKK**TSFKGDSGGPLV**CNKVAQGI**VSYGRNNGMP**

**PRAC**TKVSSFVHWIKKTMKRY

NCBI Reference Sequence. NP_004122.2, ORF21-247

FIG. 1

EP 4 159 237 A1

FIG. 2

FIG. 3

## INTERNATIONAL SEARCH REPORT

| International application No. |
|---|
| PCT/JP2021/020334 |

### A. CLASSIFICATION OF SUBJECT MATTER

Int.Cl. A61K39/395(2006.01)i, A61P29/00(2006.01)n, A61P35/00(2006.01)i, A61P43/00(2006.01)n, C12N5/10(2006.01)i, C12N1/15(2006.01)i, C12N1/19(2006.01)i, C12N1/21(2006.01)i, C12N15/12(2006.01)i, C12N15/57(2006.01)i, C12N15/63(2006.01)i, A61K38/48(2006.01)n, C12N9/64(2006.01)i, A61K35/12(2015.01)i
FI: C12N15/57, C12N15/12, C12N15/63Z, C12N1/15, C12N1/19, C12N1/21, C12N5/10, A61P35/00, A61K35/12, A61K39/395M, A61K39/395Y, C12N9/64ZNA, A61K39/395T, A61P29/00, A61P43/00105, A61K38/48

According to International Patent Classification (IPC) or to both national classification and IPC

### B. FIELDS SEARCHED

Minimum documentation searched (classification system followed by classification symbols)
Int.Cl. A61K39/395, A61P29/00, A61P35/00, A61P43/00, C12N5/10, C12N1/15, C12N1/19, C12N1/21, C12N15/12, C12N15/57, C12N15/63, A61K38/48, C12N9/64, A61K35/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII), CAplus/MEDLINE/EMBASE/BIOSIS(STN), UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | LOSASSO, V. et al., Design of human granzyme B variants resistant to serpin B9, Proteins, 2012, vol. 80, pp. 2514-2522, entire text, particularly, p. 2514, abstract, ll. 1-9, p. 2516, fig. 1, p. 2520, right column, ll. 8-23, fig. 4 | 1, 3-11<br>1, 3-23<br>2 |
| X<br>Y<br>A | WO 2013/041659 A2 (PHARMEDARTIS GMBH) 28 March 2013 (2013-03-28), entire text, particularly, claims 12, 14-16, p. 30, lines 5-9, 15-17, page 32, lines 1, 2, 11-17, fig. 6-8 | 1, 3-11<br>12-23<br>2 |

☒ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 July 2021 | 03 August 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
|---|
| PCT/JP2021/020334 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | SCHIFFER, S. et al., Efficacy of an adapted granzyme B-based anti-CD30 cytolytic fusion protein against PI-9-positive classical Hodgkin lymphoma cells in a murine model, Blood Cancer J., 2013, vol. 3, e106, pp. 1-7, entire text, particularly, p. 1, abstract, ll. 1-6, p. 4, left column, ll. 4-8, p. 4, right column, last paragraph to p. 5, left column, l. 2, fig. 4c | 1, 3-11<br>12-23<br>2 |
| X<br>Y<br>A | HEHMANN-TITT, G. et al., Improving the therapeutic potential of human granzyme B for targeted cancer therapy, Antibodies, 2013, vol. 2, pp. 19-49, entire text, particularly, p. 30, last paragraph, l. 6 to p. 31, l. 3, p. 31, ll. 5-7, 15-28, p. 46, references and note, 150 | 1, 3-11<br>1, 3-23<br>2 |
| X<br>Y<br>A | HLONGWANE, P. et al., Human granzyme B based targeted cytolytic fusion proteins, Biomedicines, 2018, vol. 6, article no. 72, pp. 1-11, entire text, particularly, p. 6, third paragraph, ll. 6-8, last paragraph, p. 7, table 3, p. 11, references, 54 | 1, 3-11<br>1, 3-23<br>2 |
| Y<br>A | GRAHAM, L. D. et al., Random mutagenesis of the substrate-binding site of a serine protease can generate enzymes with increased activities and altered primary specificities, Biochemistry, 1993, vol. 32, pp. 6250-6258, entire text, particularly, p. 6250, abstract, left column, ll. 1-17, left column, l. 25 to right column, l. 3, p. 6253, table 1, p. 6254, left column, ll. 7-11, p. 6256, right column, last paragraph to p. 6257, left column, l. 5, p. 6257, right column, ll. 22-27 | 1, 3-23<br>2 |
| Y<br>A | OBEROI, P., WELS, W. S., Arming NK cells with enhanced antitumor activity, CARs and beyond, OncoImmunology, 2013, vol. 2, no. 8, e25220, pp. 1-3, entire text, particularly, p. 1, abstract, middle column, ll. 7-14, last paragraph, ll. 1-6, right column, last paragraph, ll. 1-6, p. 2, left column, ll. 3-13, middle column, l. 4 to right column, l. 11, fig. 1C, 1D | 12-23<br>1-11 |
| Y<br>A | OBEROI, P. et al., EGFR-targeted granzyme B expressed in NK cells enhances natural cytotoxicity and mediates specific killing of tumor cells, PLoS ONE, 2013, vol. 8, no. 4, e61267, pp. 1-11, entire text, particularly, p. 1, abstract, p. 7, left column, last paragraph, l. 7 to right column, l. 2 | 12-23<br>1-11 |

Form PCT/ISA/210 (second sheet) (January 2015)

**EP 4 159 237 A1**

**INTERNATIONAL SEARCH REPORT**

| International application No. |
| --- |
| PCT/JP2021/020334 |

C (Continuation).  DOCUMENTS CONSIDERED TO BE RELEVANT

| Category* | Citation of document, with indication, where appropriate, of the relevant passages | Relevant to claim No. |
| --- | --- | --- |
| X<br>A | HE, Q. et al., Targeting cancers through TCR-peptide/MHC interactions, J. Hematol. Oncol., 2019, vol. 12, article no. 139, pp. 1-17, entire text, particularly, p. 1, abstract, p. 2, left column, ll. 21-30, p. 3, right column, ll. 8-15, p. 7, fig. 3, p. 9, right column, ll. 7-17, p. 10, fig. 4 | 18-23<br>1-17 |
| X<br>A | ANDONIOU, C. E. et al., A natural genetic variant of granzyme B confers lethality to a common viral infection, PLoS Pathog., 2014, vol. 10, no. 12, e1004526, pp. 1-13, entire text, particularly, p. 2, left column, ll. 28-34, right column, results, first paragraph, second paragraph, ll. 1-5, third paragraph, ll. 5-7, p. 3, fig. 1A, 1B | 1, 3-11<br>2, 12-23 |
| X<br>A | CULLEN, S. P. et al., Human and murine granzyme B exhibit divergent substrate preferences, J. Cell. Biol., 2007, vol. 176, no. 4, pp. 435-444, entire text, particularly, p. 436, left column, results, first paragraph, ll. 1-7, 13 to second paragraph, l. 4, p. 437, fig. 1A, p. 438, left column, ll. 7-11, p. 439, fig. 3B | 1, 3-11<br>2, 12-23 |
| X<br>A | BOTS, M. et al., Serpins prevent granzyme-induced death in a species-specific manner, Immunol. Cell. Biol., 2006, vol. 84, pp. 79-86, entire text, particularly, p. 79, summary, ll. 3-9, p. 80, right column, ll. 2-6, cell death assay, p. 81, left column, ll. 3-25, pp. 82, 83, fig. 2C, 2D and their notes | 1, 3-11<br>2, 12-23 |
| X<br>A | CASCIOLA-ROSEN, L. et al., Mouse and human granzyme B have distinct tetrapeptide specificities and abilities to recruit the bid pathway, J. Biol. Chem., 2007, vol. 282, no. 7, pp. 4545-4552, entire text, particularly, p. 4545, abstract, ll. 15, 16, p. 4546, left column, experimental procedures, first paragraph, ll. 10, 11, p. 4547, left column, ll. 4-6, p. 4548, table 1, p. 4549, left column, ll. 41-44 | 1, 3-11<br>2, 12-23 |
| X<br>A | VAN DAMME, P. et al., Analysis of protein processing by N-terminal proteomics reveals novel species-specific substrate determinants of granzyme B orthologs, Mol. Cell. Proteom., 2009, vol. 8, pp. 258-272, entire text, particularly, p. 260, left column, ll. 13-19, experimental procedures, second paragraph, ll. 1, 2, p. 265, right column, l. 5 to p. 266, left column, l. 7, fig. 5C, 5D | 1, 3-11<br>2, 12-23 |

Form PCT/ISA/210 (second sheet) (January 2015)

23

**INTERNATIONAL SEARCH REPORT**

| | International application No. |
|---|---|
| | PCT/JP2021/020334 |

C (Continuation).   DOCUMENTS CONSIDERED TO BE RELEVANT

| Category* | Citation of document, with indication, where appropriate, of the relevant passages | Relevant to claim No. |
|---|---|---|
| X<br><br>A | YEKU, O. O. et al., Armored CAR T cells enhance antitumor efficacy and overcome the tumor microenvironment, Sci. Rep., vol. 7, 2017, article no. 10541, pp. 1-14, entire text, particularly, p. 10, discussion, ll. 7-11, p. 11, T-cell isolation and gene transfer, ll. 1, 2 | 12, 13, 15-17, 23<br><br>1-11, 14, 18-22 |
| X<br><br>A | CHEN, M. et al., Antitumor efficacy of chimeric antigen receptor T cells against EGFRvIII expressing glioblastoma in C57BL/6 mice, Biomed. Pharmacother., 2019, vol. 113, article no. 108734, pp. 1-8, entire text, particularly, p. 1, right column, ll. 2-13, p. 2, left column, fourth paragraph, p. 3, left column, column 3.1, ll. 1-7, fig. 1A, right column, first paragraph, ll. 10-14, p. 4, fig. 2B | 12, 13, 15-17, 23<br><br>1-11, 14, 18-22 |
| X<br>A | DEROUAZI, M. et al., Novel cell-penetrating peptide-based vaccine induces robust CD4+ and CD8+ T cell-mediated antitumor immunity, Cancer Res., 2015, vol. 75, no. 15, pp. 3020-3031, entire text, particularly, p. 3020, abstract, p. 3021, left column, last paragraph, l. 2 to right column, l. 6, fig. 1A, p. 3027, left column, ll. 6-19 | 12-22<br>1-11, 23 |
| Y<br>A | SINGER, J., JENSEN-JAROLIM, E., IgE-based immunotherapy of cancer: challenges and chances, Allergy, 2014, vol. 69, pp. 137-149, entire text, particularly, p. 142, right column, basophils, ll. 1-3, second paragraph, ll. 1-19 | 18-22<br>1-17, 23 |
| Y<br>A | NAKAJIMA, H. et al., Synergistic roles of granzymes A and B in mediating target cell death by rat basophilic leukemia mast cell tumors also expressing cytolysin/perforin, J. Exp. Med., 1995, vol. 181, pp. 1037-1046, entire text, particularly, p. 1037, summary, ll. 1-7, p. 1038, left column, materials and methods, second paragraph, ll. 1-5 | 18-22<br>1-17, 23 |
| Y<br>A | SMITH, A. J., New horizons in therapeutic antibody discovery: opportunities and challenges versus small-molecule therapeutics, J. Biomol. Screen., 2015, vol. 20, no. 4, pp. 437-453, entire text, particularly, p. 440, right column, last paragraph, ll. 4-15, p. 439, fig. 1 | 19-22<br>1-18, 23 |

Form PCT/ISA/210 (second sheet) (January 2015)

**INTERNATIONAL SEARCH REPORT**

| International application No. |
|---|
| PCT/JP2021/020334 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2005/100556 A2 (CATALYST BIOSCIENCES) 27 October 2005 (2005-10-27), entire text, particularly, page 3, first paragraph, page 30, second paragraph, lines 2-5, table 2, page 32, last paragraph, page 33, table 4, page 42, lines 7-12, page 43, lines 21, 22, page 46, lines 3, 4, 8-10, table 5, page 80, last paragraph, lines 1-4, page 93, lines 10-31, page 96, lines 12-16, table 21, page 101, lines 27-29 | 1, 3-11<br>1, 3-11<br>2, 12-23 |
| X<br>Y<br>A | RUGGLES, S. W. et al., Characterization of structural determinants of granzyme B reveals potent mediators of extended substrate specificity, J. Biol. Chem., 2004, vol. 279, no. 29, pp. 30751-30759, entire text, particularly, p. 30751, abstract, p. 30753, fig. 1, p. 30754, table 1, p. 30755, left column, ll. 6-12 | 1, 3-11<br>1, 3-11<br>2, 12-23 |
| X<br>Y<br>A | JP 2015-532102 A (RESEARCH DEVELOPMENT FOUNDATION) 09 November 2015 (2015-11-09), entire text, particularly, claims 1, 9, 10, paragraphs [0009], [0010], [0013], [0049], fig. 1A | 1, 3-11<br>12-23<br>2 |
| X<br>A | PIUKO, K. et al., Identification and characterization of equine granzyme B, Vet. Immunol. Immunopathol., 2007, vol. 118, pp. 239-251, entire text, particularly, p. 239, abstract, p. 243, right column, last paragraph, ll. 1-13, p. 244, fig. 1 | 1, 3-11<br>2, 12-23 |
| X<br>A | FU, Z. et al., Asp-ase activity of the opossum granzyme B supports the role of granzyme B as part of anti-viral immunity already during early mammalian evolution, PLoS ONE, 2016, vol. 11, no. 5, e0154886, pp. 1-20, entire text, particularly, p. 1, abstract, p. 9, first paragraph, second paragraph, ll. 7-14, p. 18, supporting information, first paragraph, fig. S1 | 1, 3-11<br>2, 12-23 |
| X<br>A | ERICSEN, A. J. et al., Whole genome sequencing of SIV-infected macaques identifies candidate loci that may contribute to host control of virus replication, Genome Biology, 2014, vol. 15, article no. 478, pp. 1-14, entire text, particularly, p. 1, abstract, p. 12, right column, second paragraph, ll. 5-13 | 1, 3-11<br>2, 12-23 |
| X<br>A | Macaca fascicularis granzyme B (GzmB) gene, GzmB-1:01:01 allele, complete cds, GenBank, Accession no. KM281203.1, 13 November 2014, [Retrieved on 01 July 2021], Retrieved from the Internet, URL, <https://www.ncbi.nlm.nih.gov/nuccore/KM281203.1?report=girevhist>, entire text | 1, 3-11<br>2, 12-23 |

Form PCT/ISA/210 (second sheet) (January 2015)

**INTERNATIONAL SEARCH REPORT**

| International application No. |
| --- |
| PCT/JP2021/020334 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>A | PREDICTED: granzyme B [Propithecus coquereli], NCBI Ref. Seq., Accession no. XP—012502759.1, 01 June 2015, [retrieved on 06 July 2021], Retrieved from the Internet, URL, <https://www.ncbi.nlm.nih.gov/protein/XP_012502759.1?report=girevhist>, entire text | 1, 3-11<br>2, 12-23 |
| X<br>A | Granzyme B [Tupaia chinensis], GenBank, Accession no. ELW69303.1, 19 March 2015, [Retrieved on 05 July 2021], Retrieved from the Internet, URL, <https://www.ncbi.nlm.nih.gov/protein/ELW69303.1?report=girevhist>, entire text | 1, 3-11<br>2, 12-23 |
| X<br>A | Granzyme B isoform X1 [Physeter catodon], NCBI Ref. Seq., Accession no. XP_007119743.1, 21 March 2019, [Retrieved on 05 July 2021], Retrieved from the Internet, URL, <https://www.ncbi.nlm.nih.gov/protein/XP_007119743.1?report=girevhist>, entire text | 1, 3-11<br>2, 12-23 |
| X<br>A | Granzyme B-like isoform XI [Delphinapterus leucas], NCBI Ref. Seq., Accession no. XP_022409388.1, 16 September 2019, [retrieved on 05 July 2021], Retrieved from the Internet, URL, <https://www.ncbi.nlm.nih.gov/protein/XP_022409388.1?report=girevhist>, entire text | 1, 3-11<br>2, 12-23 |
| X<br>A | Granzyme B-like isoform XI [Neophocaena asiaeorientalis asiaeorientalis], NCBI Ref. Seq., Accession no. XP_024624959.1, 18 April 2018, [retrieved on 05 July 2021], Retrieved from the Internet, URL, <https://www.ncbi.nlm.nih.gov/protein/XP_024624959.1?report=girevhist>, entire text | 1, 3-11<br>2, 12-23 |
| X<br>A | PREDICTED: granzyme B [Lipotes vexillifer], NCBI Ref. Seq., Accession no. XP_007471896.1, 01 April 2014, [retrieved on 05 July 2012], Retrieved from the Internet, URL, <https://www.ncbi.nlm.nih.gov/protein/XP_007471896.1?report=girevhist>, entire text | 1, 3-11<br>2, 12-23 |
| X<br>A | Granzyme B [Tursiops truncatus], NCBI Ref. Seq., Accession no. XP_033708488.1, 27 April 2020, [retrieved on 05 July 2021], Retrieved from the Internet, URL, <https://www.ncbi.nlm.nih.gov/protein/XP_033708488.1?report=girevhist>, entire text | 1, 3-11<br>2, 12-23 |

Form PCT/ISA/210 (second sheet) (January 2015)

**INTERNATIONAL SEARCH REPORT**

| International application No. |
| --- |
| PCT/JP2021/020334 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>A | PREDICTED: granzyme B-like [Bison bison bison], NCBI Ref. Seq., Accession no. XP_010841689.1, 31 December 2014, [retrieved on 05 July 2021], Retrieved from the Internet, URL, <https://www.ncbi.nlm.nih.gov/protein/XP_010841689.1?report=girevhist>, entire text | 1, 3-11<br>2, 12-23 |
| X<br>A | PREDICTED: granzyme B [Capra hircus], NCBI Ref. Seq., Accession no. XP_017900778.1, 08 September 2016, [Retrieved on 05 July 2021], Retrieved from the Internet, URL, <https://www.ncbi.nlm.nih.gov/protein/XP_017900778.1?report=girevhist>, entire text | 1, 3-11<br>2, 12-23 |
| X<br>A | Granzyme B-like [Ovis aries], NCBI Ref. Seq., Accession no. XP_027813308.1, 06 February 2019, [retrieved on 05 July 2021], Retrieved from the Internet, URL, <https://www.ncbi.nlm.nih.gov/protein/XP_027813308.1?report=girevhist>, entire text | 1, 3-11<br>2, 12-23 |
| X<br>A | Granzyme B, partial [Bos mutus], GenBank, Accession no. ELR49680.1, 19 March 2015, [retrieved on 05 July 2021], Retrieved from the Internet, URL, <https://www.ncbi.nlm.nih.gov/protein/ELR49680.1?report=girevhist>, entire text | 1, 3-11<br>2, 12-23 |
| X<br>A | Granzyme B [Camelus dromedarius], GenBank, Accession no. KAB1278066.1, 04 October 2019, [retrieved on 06 July 2021], Retrieved from the Internet, URL, <https://www.ncbi.nlm.nih.gov/protein/KAB1278066.1?report=girevhist>, entire text | 1, 3-11<br>2, 12-23 |
| X<br>A | Granzyme B, UniProt, Accession no. A0A2F0B3Z3, 22 April 2020, [retrieved on 05 July 2021], Retrieved from the Internet, URL, <https://www.uniprot.org/uniprot/A0A2F0B3Z3?version=*>, entire text | 1, 3-11<br>2, 12-23 |
| X<br>A | PREDICTED: granzyme B-like [Dipodomys ordii], NCBI Ref. Seq., Accession no. XP_012880796.1, 26 June 2015, [retrieved on 05 July 2021], Retrieved from the Internet, URL, <https://www.ncbi.nlm.nih.gov/protein/XP_012880796.1?report=girevhist>, entire text | 1, 3-11<br>2, 12-23 |

Form PCT/ISA/210 (second sheet) (January 2015)

**INTERNATIONAL SEARCH REPORT**

| International application No. |
| --- |
| PCT/JP2021/020334 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>A | PREDICTED: granzyme B-like [Dipodomys ordii], NCBI Ref. Seq., Accession no. XP_012880811.1, 26 June 2015, [retrieved on 06 July 2021], Retrieved from the Internet, URL, <https://www.ncbi.nlm.nih.gov/protein/XP_012880811.1?report=girevhist>, entire text | 1, 3-11<br>2, 12-23 |
| X<br>A | Granzyme B, UniProt, Accession no. A0A2K5YY00, 26 February 2020, [retrieved on 01 July 2021], Retrieved from the Internet, URL, <https://www.uniprot.org/uniprot/A0A2K5YY00?version=*>, entire text | 1, 3-11<br>2, 12-23 |
| X<br>A | PREDICTED: granzyme B [Cercocebus atys], NCBI Ref. Seq., Accession no. XP_011946523.1, 30 March 2015, [retrieved on 01 July 2021], Retrieved from the Internet, URL, <https://www.ncbi.nlm.nih.gov/protein/XP_011946523.1?report=girevhist>, entire text | 1, 3-11<br>2, 12-23 |
| X<br>A | Granzyme B [Aotus nancymaae], NCBI Ref. Seq., Accession no. XP_012323554.1, 28 June 2017, [retrieved on 05 July 2021], Retrieved from the Internet, URL, <https://www.ncbi.nlm.nih.gov/protein/XP_012323554.1?report=girevhist>, entire text | 1, 3-11<br>2, 12-23 |
| X<br>A | Granzyme B [Saimiri boliviensis boliviensis], NCBI Ref. Seq., Accession no. XP_003924294.2, 24 November 2014, [retrieved on 05 July 2021], Retrieved from the Internet, <https://www.ncbi.nlm.nih.gov/protein/XP_003924294.2?report=girevhist>, entire text | 1, 3-11<br>2, 12-23 |
| X<br>A | Granzyme B isoform XI [Gorilla gorilla gorilla], NCBI Ref. Seq., Accession no. XP_004055081.1, 30 September 2019, [retrieved on 01 July 2021], Retrieved from the Internet, URL, <https://www.ncbi.nlm.nih.gov/protein/XP_004055081.1?report=girevhist>, entire text | 1, 3-11<br>2, 12-23 |
| X<br>A | Granzyme B [Trichechus manatus latirostris], NCBI Ref. Seq., Accession no. XP_004376613.1, 25 January 2018, [retrieved on 05 July 2021], Retrieved from the Internet, URL, <https://www.ncbi.nlm.nih.gov/protein/XP_004376613.1?report=girevhist>, entire text | 1, 3-11<br>2, 12-23 |

Form PCT/ISA/210 (second sheet) (January 2015)

**INTERNATIONAL SEARCH REPORT**

International application No.

PCT/JP2021/020334

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | YANG, J. et al., Granzyme B is an essential mediator in CD8+ T cell killing of theileria parva-infected cells, Infect. Immun., 2018, vol. 87, no. 1, e00386-18, pp. 1-15, entire text, particularly, p. 1, abstract, p. 3, last paragraph, l. 3 to p. 4, l. 2, fig. 1 | 1, 3-11<br>2, 12-23 |
| P, X<br>P, A | WO 2020/169620 A1 (ATB THERAPEUTICS) 27 August 2020 (2020-08-27), entire text, particularly, claim 15, pages 53, 54, sequence no. 17 | 1, 3-11<br>2, 12-23 |
| A | WO 2006/026451 A2 (CHILDREN'S HOSPITAL INC.) 09 March 2006 (2006-03-09), entire text, particularly, claims 12, 13, page 8, lines 8-13, page 18, table 1 | 1-23 |
| A | WO 2017/143026 A1 (RESEARCH DEVELOPMENT FOUNDATION) 24 August 2017 (2017-08-24), entire text, particularly, claims 1, 3, 5, paragraphs [0077], [0078], [0082] | 1-23 |

Form PCT/ISA/210 (continuation of second sheet) (January 2015)

| INTERNATIONAL SEARCH REPORT | International application No. |
|---|---|
| | PCT/JP2021/020334 |

**Box No. II    Observations where certain claims were found unsearchable (Continuation of item 2 of first sheet)**

This international search report has not been established in respect of certain claims under Article 17 (2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**

International application No.

PCT/JP2021/020334

**Box No. III** **Observations where unity of invention is lacking (Continuation of item 3 of first sheet)**

This International Searching Authority found multiple inventions in this international application, as follows:

The inventions set forth in the claims are classified into the following 33 inventions.

(1): Parts of the inventions in claims 1, 3-11, 15-17 and 20-23, wherein "one or more amino acid residues" in the "granzyme B variant" contain "1) T at position 43", entire claims 2, 12-14, 18 and 19 and another parts of claims 3-11, 15-17 and 20-23; and

(2)-(33): rest of claims 1, 3-11, 15-17 and 20-23, namely each of 32 parts in claims 1, 3-11, 15-17 and 20-23, wherein "one or more amino acid residues" in the "granzyme B variant" contain another amino acid at the same position as or different position from "1) T at position 43" (provided that all are such that "one or more amino acid residues" do not contain "1) T at position 43" and all do not contain "a combination of any amino acid residues of 1-12" in claim 2).

Form PCT/ISA/210 (continuation of first sheet) (January 2015)

| INTERNATIONA1 SEARCH REPORT | InternationA1 application No. |
| Information on patent family members | PCT/JP2021/0203342021/0 |

| | | |
|---|---|---|
| WO 2013/041659 A2 | 28 March 2013 | US 2014/0356347 A1<br>entire text<br>EP 2758527 A2 |
| WO 2005/100556 A2 | 27 October 2005 | EP 1735438 A2<br>entire text |
| JP 2015-532102 A | 09 November 2015 | WO 2014/055836 A2<br>entire text, particularly,<br>claims 1, 9, 10, paragraphs [0009],<br>[0010], [0013], [0049],<br>fig. 1A<br>EP 2904004 A2<br>US 2014/0314760 A1 |
| WO 2020/169620 A1 | 27 August 2020 | (Family: none) |
| WO 2006/026451 A2 | 09 March 2006 | US 2009/0239937 A1<br>entire text |
| WO 2017/143026 A1 | 24 August 2017 | US 2017/0260517 A1<br>entire text<br>EP 3417058 A1 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9528101 B
- WO 2005100556 A2
- JP 2019526285 A

**Non-patent literature cited in the description**

- **LUIS MARTINEZ-LOSTAO, ALBERTO ANEL AND JULIAN PARDO.** How Do Cytotoxic Lymphocytes Kill Cancer Cells?. *Clin Cancer Res.,* 15 November 2015, vol. 21 (22), 5047-56
- **KURSCHUS FC1, JENNE DE.** Delivery and therapeutic potential of human granzyme B. *Immunol Rev.,* May 2010, vol. 235 (1), 159-71
- **J. P. MEDEMA ; J. DE JONG ; L. T. C. PELTENBURG ; E. M. E. VERDEGAAL ; A. GORTER ; S. A. BRES ; K. L. M. C. FRANKEN ; M. HAHNE ; J. P. ALBAR ; C. J. M. MELIEF.** Blockade of the granzyme B/perforin pathway through overexpression of the serine protease inhibitor PI-9/SPI-6 constitutes a mechanism for immune escape by tumors. *Proc Natl Acad Sci USA.,* 25 September 2001, vol. 98 (20), 11515-11520
- **CATHERINA H. BIRD, JIURU SUN, KHENG UNG, DIANA KARAMBALIS, JAMES C. WHISSTOCK, JOSEPH A. TRAPANI, AND PHILLIP I. BIRD.** Cationic Sites on Granzyme B Contribute to Cytotoxicity by Promoting Its Uptake into Target Cells. *Mol Cell Biol.,* September 2005, vol. 25 (17), 7854-7867
- **LOSASSO V, SCHIFER S, BARTH S, CARLONI P.** Design of human granzyme B variants resistant to serpin B9. *Proteins,* November 2012, vol. 80 (11), 2514-22
- **KURSCHUS FC, FELLOWS E, STEGMANN E, JENNE DE.** Granzyme B delivery via perforin is restricted by size, but not by heparan sulfate-dependent endocytosis. *Proc Natl Acad Sci USA.,* 16 September 2008, vol. 105 (37), 13799-13804
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- *J Vis Exp.,* 10 June 2015, vol. 100, e52911
- *Protein Science,* 1995, vol. 4, 2411-2423
- **JOHNSON et al.** *Blood,* 2009, vol. 114, 535-546